# EUROPÄISCHE PATENTSCHRIFT

(11) **EP 1 514 545 B1**
(45) Veröffentlichungstag und Bekanntmachung des Hinweises auf die Patenterteilung: **23.01.2008**
(21) Anmeldenummer: 04020218.6
(22) Anmeldetag: 26.08.2004
(51) Int. Cl.: A61K 31/4453, A61P 1/06, A61P 11/06, A61P 15/00

(54) **Verwendung von Tolperison zur Behandlung von Spasmen von Organen, die im wesentlichen aus glatter Muskulatur aufgebaut sind.**
Use of tolperisone for the treatment of spasms of organes consisting essentially of smooth muscles.
Utilisation de tolperison pour le traitement d' organes essentiellement formés de muscles lisses

(30) Priorität: 11.09.2003 DE 10341923
(43) Veröffentlichungstag der Anmeldung: 16.03.2005
(73) Patentinhaber: Heinl, Alexander, Dr. med., 55118 Mainz (DE)
(72) Erfinder: Heinl, Alexander, Dr. med., 55118 Mainz (DE)
(74) Vertreter: Pöhner, Wilfried Anton, Dr.

(56) Entgegenhaltungen:
- EP-A- 0 273 375
- GB-A- 1 452 868
- FURUTA Y ET AL: "Effect of tolperisone on the in situ blood-perfused dog trachea" JAPANESE JOURNAL OF PHARMACOLOGY 1977 JAPAN, Bd. 27, Nr. suppl., 1977, Seite 71P, XP009041118
- ANON: "1-4 ETHYLPHENYL-2-METHYL-3-1-PIPERIDINYL-1 PROPANONE HYDRO CHLORIDE" DRUGS OF THE FUTURE, Bd. 7, Nr. 2, 1982, Seiten 105-106, XP009041115 ISSN: 0377-8282
- BECK L ET AL: "Back pain in gynaecological practice" GYNAKOLOGE 2002 GERMANY, Bd. 35, Nr. 5, 2002, Seiten 490-494, XP009041117 ISSN: 0017-5994
- NAGAI H ET AL: "Immunopharmacological studies on mydocalm and related compounds as an antagonist of slow reacting substance of anaphylaxis (SRS-A)" JOURNAL OF PHARMACOBIO-DYNAMICS 1983 JAPAN, Bd. 6, Nr. 11, 1983, Seiten 874-880, XP009041116

## Beschreibung

Die Erfindung betrifft die Verwendung von Tolperison sowie von dessen physiologisch verträglichen Säureadditionsalzen in Kombination mit üblichen Hilfs- und/oder Trägerstoffen zur Herstellung eines Arzneimittels gegen schmerzhafte Spasmen, Muskelverspannungen und/oder Erkrankungen mit Symptomen im muskulären Bereich von Organe, die im wesentlichen aus glatter Muskulatur aufgebaut sind.

Tolperison ist der internationale Freiname für die als Muskelrelaxans eingesetzte Substanz (RS)-2,4'-Dimethyl-3-piperidinopropiophenon mit der Summenformel C₁₆H₂₃NO. Das Muskelrelaxans hat als Indikationsgebiet die ambulante Behandlung akuter oder chronisch spastischer und hypertoner Zustände der quergestreiften Skelettmuskulatur. Tolperison wird auch eingesetzt bei schmerzhaftem Muskelhartspann infolge von degenerativen Veränderungen der Wirbelsäule. Der Wirkmechanismus von Muskelrelaxantien ist im Detail noch nicht verstanden.

Aus der US 1 452 868 ist bekannt, dass die chemische Verbindung Tolperison sich als Therapeutikum zur Behandlung krampfartiger Lähmungen (Spastic Paralysis) eignet. Hierbei werden als Wirkmechanismen die Reflexunterdrückung in den Synapsen der Wirbelsäule angegeben. Auch in der EP 0 273 375 wird die muskelrelaxierende Wirkung von Tolperison genannt.

Die laufenden Forschungsarbeiten und die durch Schutzrechte geschützten Weiterentwicklungen des Wirkstoffs betreffen Propiophenonderivate und insbesondere die Verbesserung der pharmazeutischen Zubereitung von Tolperison. So ist z.B. in AT 409083 B ein Verfahren zur Herstellung verschiedener pharmazeutischer Zubereitungen von Tolperison zur oralen Verabreichung beschrieben, durch die eine verzögerte Freisetzung des Wirkstoffs im Blut erreicht werden soll, so dass der gewünschte Gehalt an Tolperison über eine vergleichsweise lange Zeitspanne aufrecht erhalten werden kann.

Beim Muskelgewebe unterscheidet man zwischen quergestreifter (Skelett-) Muskulatur, glatter Muskulatur und der Herzmuskulatur, die eine Sonderform der quergestreifen Muskulatur darstellt.

Die quergestreifte Muskulatur weist langgestreckte quergestreifte Fasern mit einer Vielzahl elliptischer randständiger Kerne auf. Die Muskelfibrillen der Muskelfasem sind in kontraktile Einheiten gegliedert, die aus ineinanderliegenden Myosin- und Actinsträngen aufgebaut werden. Die glatte Muskulatur weist spindelförmige Muskelzellen mit längsovalem, zentral gelegenem Zellkern auf, die räumlich netzförmig angeordnet oder in Schichten gepackt sind. Im Gegensatz zu den beiden anderen Muskelgeweben sind Kontraktion und Entspannung der quergestreiften Muskulatur dem menschlichen Willen unterworfen. Die unwillkürlich arbeitende glatte Muskulatur ist bei Wirbeltieren besonders vom vegetativen Nervensystem, das nicht dem Bewußtsein untergeordnet ist und der Regelung der Vitalfunktionen (z. B. Atmung, Verdauung, Stoffwechsel, Sekretion) dient, inerviert und findet sich in der Wandung innerer Hohlorgane wie z. B. Magen, Darm, Harnblase, Harnleiter und im Uterus sowie in den Luftwegen, aber auch in Lymph- und Blutgefäßen, sowie in der Iris im Auge vorkommt. Glatte Muskelzellen zeichnen sich durch ihr hohes Kontraktionsvermögen und geringe Ermüdbarkeit bei geringerer Kontraktionskraft und Kontraktionsgeschwindigkeit aus. Diese Besonderheiten zeigen, dass es sich in den Augen des medizinisch geschulten Fachmanns bei der glatten Muskulatur um ein anderes Gewebe handelt, als bei der quergestreiften Muskulatur.

Gegenstand der vorliegenden Erfindung ist die Verwendung von Tolperison sowie der physiologisch verträglichen Säureadditionsalzen von Tolperison zur Herstellung von Arzneimitteln zur Behandlung von Spasmen und Muskelverspannungen der zum Uterus gehörenden glatten Muskulatur insbesondere während der Menstruation und bei Vorliegen der medizinischen Indikation der primären und/oder sekundären Dysmenorrhoe. Dabei handelt es sich um eine über die normale Beeinträchtigung des Allgemeinbefindens hinausgehende Schmerzhaftigkeit der Menstruation aufgrund organischer oder funktioneller Störungen.

Die Verwendung von Tolperison als Arzneimittel zur Behandlung der primären und sekundären Endometriose liegt ebenfalls im Rahmen der Erfindung. Bei der Endometriose handelt es sich um ein ortsfremdes Auftreten von funktionstüchtiger, sich unter zyklischen Blutungen abstoßenden Gebärmutterschleimhaut im Körper. Die primäre Endometriose ist mit Tiefenwucherungen in der Gebärmuttermuskulatur verbunden. Durch Verschleppung der Schleimhaut mit der Lymphflüssigkeit in die Eileiter, die Eierstöcke, die freie Bauchhöhle und in seltenen Fällen in die Blase, den Darm oder die Lunge kann es zur Auslösung einer sekundären Endometriose kommen.

Die Endometriose führt in der Gebärmutter und den anderen befallenen Organsystemen verstärkt zu schmerzhaften Menstruationen (Dysmenorrhoe), die sich nach außen durch starke Spannungs- und Einengungsbeschwerden deutlich machen.

## Patentansprüche

1. Verwendung von Tolperison sowie von dessen physiologisch verträglichen Säureadditionsalzen in Kombination mit üblichen Hilfs- und/oder Trägerstoffen zur Herstellung eines Arzneimittels gegen schmerzhafte Spasmen, Muskelverspannungen und/oder Erkrankungen mit Symptomen im muskulären Bereich von Organen, die im wesentlichen aus glatter Muskulatur aufgebaut sind, **dadurch gekennzeichnet, dass** es sich um schmerzhafte Spasmen und Muskelverspannungen der glatten Muskulatur des Uterus handelt

2. Verwendung von Tolperison sowie von dessen physiologisch verträglichen Säureadditionsalzen in Kombination mit üblichen Hilfs- und/oder Trägerstoffen zur Herstellung eines Arzneimittels nach Anspruch 1, **dadurch gekennzeichnet, dass** es sich um Beschwerden der primären und/oder sekundären Dysmenorrhoe handelt.

3. Verwendung von Tolperison sowie von dessen physiologisch verträglichen Säureadditionsalzen in Kombination mit üblichen Hilfs- und/oder Trägerstoffen zur Herstellung eines Arzneimittels nach Anspruch 1 oder 2, **dadurch gekennzeichnet, dass** es sich um Beschwerden der Endometriose handelt.

## Claims

1. Use of tolperisone and its physiologically compatible acid addition salts in combination with conventional additives and/or excipients for producing a pharmaceutical against painful spasms, muscle tension and/or illnesses with symptoms in the muscle area of organs that are essentially made up of smooth muscle **characterised in that** painful spasms and muscular tensions of the smooth muscle of the uterus are concerned.

2. Use of tolperisone and its physiologically compatible acid addition salts in combination with conventional additives and/or excipients for producing a pharmaceutical according to claim 1, **characterised in that** complaints of the primary and/or secondary dysmenorrhoea are concerned.

3. Use of tolperisone and its physiologically compatible acid addition salts in combination with conventional additives and/or excipients for producing a pharmaceutical according to claim 1 or 2, **characterised in that** complaints of endometriosis are concerned.

## Revendications

1. Utilisation de tolpérisone ainsi que de ses sels additionnels physiologiquement supportables en combinaison avec des agents auxiliaires et/ou porteurs pour fabriquer un médicament contre les spasmes douloureux, les tensions musculaires et/ou les maladies présentant des symptômes dans la zone musculaire d'organes essentiellement constitués de muscles lisses, **caractérisée par le fait qu'**il s'agit de spasmes douloureux et de tensions des muscles lisses de l'utérus.

2. Utilisation de tolpérisone ainsi que de ses sels additionnels physiologiquement supportables en combinaison avec des agents auxiliaires et/ou porteurs pour fabriquer un médicament selon la revendication 1, **caractérisée par le fait qu'**il s'agit de troubles de la dysménorrhée primaire et/ou secondaire.

3. Utilisation de tolpérisone ainsi que de ses sels additionnels physiologiquement supportables en combinaison avec des agents auxiliaires et/ou porteurs pour fabriquer un médicament selon les revendications 1 ou 2, **caractérisée par le fait qu'**il s'agit de troubles d'endométriose
